# EUROPEAN PATENT APPLICATION

(11) **EP 2 853 573 A1**
(43) Date of publication of application: **01.04.2015**
(21) Application number: 13198936.0
(22) Date of filing: 20.12.2013
(51) Int. Cl.: C09J 167/02, A61F 13/02, C09J 175/06

(54) **Skin friendly adhesive**

(30) Priority: 25.09.2013 EP 13185924
(71) Applicant: NITTO DENKO CORPORATION, Osaka 567 (JP)
(72) Inventor: Schüwer, Nicolas, 1007 Lausanne (CH); Takeiri, Mayu, 1025 St. Sulpice (CH); Higashi, Masatsugu, Aichi-ken 440-0038 (JP); Vendamme, Richard, 3500 Hasselt (BE)
(74) Representative: Hoffmann Eitle

(57) **Abstract**

The present invention provides an adhesive composition, medical tape and patch as well as methods for manufacturing the same, wherein said products allow to combine excellent skin friendliness with satisfactory adhesion. The adhesive composition of the present invention is characterized by the presence of a polycondensate having a specified molecular weight, and an oil additive in a specified relative amount.

## Description

### 1. Technical Field of the Invention

This invention relates to a skin-friendly pressure sensitive adhesive. It also pertains to products containing the same, such as skin-friendly medical tapes and skin-friendly patches. The invention further pertains to methods for manufacturing these products.

### 2. State of the art

US 2010/003312 A1 relates an external skin patch with sufficient adherence but that can be easily removed. The acrylic adhesive is loaded with 0.1 to 20 wt % of dextrin fatty acid ester in order to improve the tack and the peel properties.

US 2010/0022614 A1 relates a pharmaceutical composition for external application and adhesive skin patch, in which the adhesive - based on a styrene-isoprene-styrene block copolymer - may contain between 0.1 to 20 wt % of hydrogenated oil. The role played by the hydrogenated oil is not explained.

US 2008/0032127 A1 relates to a pressure sensitive adhesive composition and skin patch which has controlled pressure-sensitive adhesiveness, cohesive force and favorable properties for skin application. More specifically the acrylic based adhesive reported contains a fatty acid derivative as plasticizer (from 30 to 200 parts relative to 100 parts by weight of polymer), which reduces the skin irritation property.

US 2007/0077285 A1 relates to an adhesive skin patch composed of a (meth)acrylic copolymer and a resolvent (aliphatic alcohol bearing a branched-chain structure) and a plasticizer that can be a derivative from natural oil.

US 2010/0056972 A1 relates to an adhesive patch. In a preferred embodiment the adhesive is made of high molecular weight polyisobutylene loaded with glycols, fat, oil, glycerol esters or surfactants.

US 2007/0004896 A1 relates to an active energy radiation hardenable skin plaster composition and skin plaster that contains an oil component (such as fatty acids derivatives). The oil additive improves skin adhesion and prevents skin damage during peel off.

WO 2011/023255 A1 relates to a bio-based adhesive using a cross-linked dimer fatty acid - dimer diol based polymer; however the crosslinking technology reported in this patent may not be the most desired for biomedical applications since it requires the use of a catalyst such as 1,8-Diazabicyclo[5.4.0]undec-7-ene that may have undesired biological effects.

All the adhesive formulation mentioned above rely on organic based polymers; on the other hand silicone adhesives are also widely employed as skin friendly adhesives. The cost associated with silicone based product together with the more complex chemical process required for the synthesis of silicon based medical tape are the main drawback of those materials. Identically, using silicone adhesives also increase production cost due to their handling difficulties.

### 3. Problem solved by the invention

Having regard to the state of the art summarized above, there remains a need for skin adhesives and patches containing the same, which simultaneously provide satisfactory adhesiveness and skin friendliness both during use and at the time of removing the patch. It is also the aim of the present invention to produce a skin-friendly adhesive that possesses equivalent or enhanced properties as compared to already known adhesives (silicon or acrylic based) but at advantageous production and raw material costs.

### 4. Summary of the Invention

The present invention meets this demand as it provides skin friendly adhesives, so-called pressure sensitive adhesive, that possess enhanced properties as compared to already known skin adhesives. The skin friendliness is the primary target, and the adhesive is furthermore characterized by a high level adhesiveness. Additionally, it is preferably made by using as much as possible of biobased materials. The specificity of the adhesive described within this document lies in its properties, namely the coexistence of high tack and skin-friendliness.

In particular, the above objectives are accomplished by means of the adhesive specified in appended claim 1. Preferred embodiments of the adhesive of the present invention are specified in appended dependent claims 2 to 8.

In another aspect of the present invention, methods are provided for manufacturing the adhesive of the invention. This aspect is specified in appended claim 9. A preferred embodiment of this aspect is characterized in appended dependent claim 10.

In yet another aspect of the present invention, a medical tape or patch comprising the adhesive of the invention is provided. This aspect is specified in appended claim 11.

In a final aspect of the present invention, methods are provided for manufacturing the medical tape of the invention and the patch of the invention. These methods are specified in appended claims 12 and 13.

### 5. Detailed Description of the Invention

### 5.0 Description of Figure

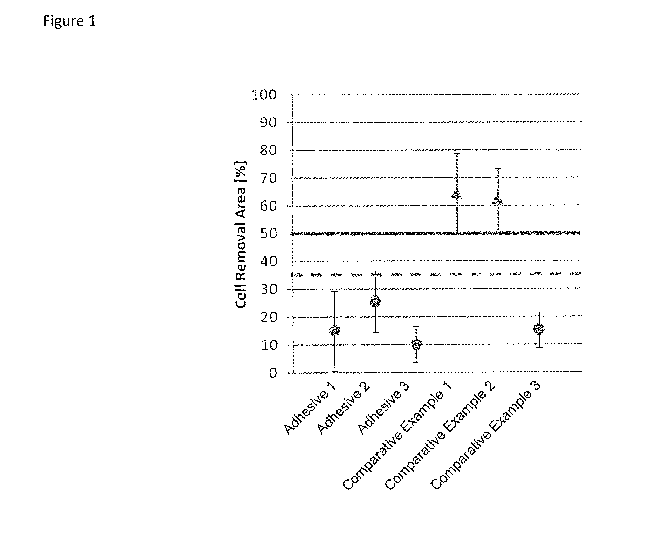

Figure 1 shows the cell removal areas experimentally determined for the inventive and comparative adhesive compositions in the examples.

### 5.1 Definitions

The expression ***"skin friendliness"*** is meant to characterize adhesives for which:
1) a very low amount of skin cells is found once the adhesive is peeled from the skin (i.e. low cell removal area value).
2) the force required to peel off the adhesive from the skin do not generate pain to the subject it is applied on.

The skin-friendliness can be assessed by means of the following methods "Peel Test on human skin": Healthy volunteer subjects (male and female) are enrolled into this study. The arms of the subjects are wiped using ethanol prior to sample application. Samples of given size are placed on the subject's arm (the spot of application of a given sample is randomized on each subject and each adhesive is tested several time on different skin area). The sample materials are secured using a 2 kg roller and allowed for 30 min before removal. The samples are peeled off the subject arm at 180 degrees at a rate of 300mm/min. The peel force is measured using a tensile testing machine and sensation of pain is recorded. "Cell Removal evaluation": A removed adhesive tape from human skin is soaked in paraformaldehyde solution in PBS. Next it is rinsed with distilled water, and the tape is incubated with 1% eosin solution in order to stain it. Then the tape is washed by distilled water and observed by microscope. An adhesive is considered to be skin friendly if it gives rise to less than 50% cell removal area, preferably less than 35% cell removal area. The results of these tests may be combined with biological evaluation (such as toxicity, sensitization and irritation assessment). Subjective factors such as itchiness, comfort of the adhesive when applied on the skin or the pain experienced upon peeling off the adhesive are also factors to be taken into account. If the peel test on human skin cannot be carried out, a corresponding test using porcine skin, as described in the examples section, may be used instead to evaluate skin friendliness. For this test, the same criteria of evaluation apply. In case of conflicting results, the results of the peel test on human skin are authoritative.

Furthermore in order to immobilize objects on the skin such as gauze, catheters and so on, the medical tape needs to have high adhesiveness on skin. On the other hand, the medical tape is also required to create low damage to human body upon peeling and when in contact with the skin. These two properties of are difficulty compatible, but the present invention offers good performance on those two aspect.

The term ***"adhesive gel content value"*** refers to the value determined by the known method reported earlier (Vendamme R. and Eevers W. Macromolecules, 2013, 46 (9), 3395-3405).

The term ***"α transition"*** refers to the value determined by rheology. Rheological measurement were performed on a Discovery HR-2 hybrid rheometer (TA Instruments) in oscillation mode using parallel plate geometry. The α transition was measured by monitoring tan α by scanning the temperature from -100°C to 150°C (ramp of 5 K/min) while imposing a constant strain of 0.4% and at a frequency of 1 Hz (linear regime conditions).

The term ***"peel force"*** or ***"adhesion level"*** refers to the force required to remove the tape after applying it to human skin and leaving it on the skin for 30 minutes (unless specified otherwise). It can be measured using a Shimadzu Ag-X universal tensile testing machine. Again, porcine skin may be used as a substitute if testing on human skin is not available. In case of doubt, human skin test results are authoritative.

Unless specified otherwise, references to the ***molecular weight*** of a polymeric substance are to be understood as references to the weight average molecular weight, which is determined by GPC using Shimadzu GPC system equipped with a differential refractive index detector and calibrated with polystyrene standards.

Unless specified otherwise, amount ***indications in %*** are to be understood as indications in weight %. Indications in wt.% *"relative to"* another compound or relative to the weight of another compound are meant to characterize the (weight of the compound of interest) divided by the (weight of the specified another compound) multiplied with 100%

The term ***"fatty acid"*** is meant to refer to carboxylic acids having a long aliphatic chain, which may be saturated or may contain one or more unsaturations. The aliphatic chain may range from 4 to 30 carbon atoms, preferably from 6 to 28 carbon atoms, more preferably from 8 to 25 carbon atoms and even more preferably from 10 to 22 carbon atoms.

The term ***"fatty alcohol"*** is meant to refer to an aliphatic alcohol having a long aliphatic chain, which may be saturated or may contain one or more unsaturations. The aliphatic chain may range from 4 to 30 carbon atoms, preferably from 6 to 28 carbon atoms, more preferably from 8 to 25 carbon atoms and even more preferably from 10 to 22 carbon atoms.

The term ***"bio-based"*** is meant to refer to substances derived from renewable starting materials (as opposed to mineral oil-derived starting materials) by isolation and optionally one or more chemical transformations.

***"Suitability for topical application to the human skin"*** means in the context of the present application that the substance of interest must be authorized by European regulatory bodies for incorporation into medical preparations that are to be applied to the human skin. The final adhesive composition must be suitable for topical application to the human skin. This means that the cross-linked polycondensate, the oil additive and any possibly present additional components must be suitable for application to human skin. However, it does not mean that individual raw materials must be suitable for application to human skin if these are consumed completely during the manufacturing process, or if residual raw material remains only at acceptably low levels or the product can be purified such that the purified product contains no or only acceptably low levels of potentially harmful raw materials.

In the context of the present application, the term ***"comprising"*** is meant to introduce an open list, such that further unmentioned members are not excluded. However, the use of "comprising" is intended to specifically characterize also the scenario, wherein no further unmentioned members are present. In other words, a disclosure of "comprising" is to be understood as a disclosure of "consisting of" as one distinct option.

In the context of the present application, the reference to a feature being a ***"preferred"*** feature is meant to indicate that it is preferred to combine this feature with other preferred features of the present application. All such combinations of preferred features with other preferred features are meant to be directly and unambiguously disclosed.

In the context of the present application, multiple limitations are to be considered in a cumulative manner. If, for instance, there should be a diacid component (a) that is within the scope of the general formula (I) shown below, but which nevertheless has high toxicity such that even trace amounts thereof should not be tolerable, its use would not be in accordance with the present invention.

### 5.2 Polycondensate (A)

### 5.2.1 Raw materials

The polycondensate (A) reported herein is obtained by reaction of raw materials comprising at least one diacid (a) and at least one diol (b). Optional additional components (c) may also be present.

### Diacid (a):

The diacids suitable for use in the present invention are characterized by the following general formula (I):

R^{a}OOC-R¹-R²-R³-COOR^{b} (I)

wherein R¹ is absent or selected from C₁₋₁₅ alkylene, C₂₋₁₅ alkenylene, and C₂₋₁₅ alkynylene; R² is selected from C₁₋₁₂ alkylene, C₂₋₁₂ alkenylene, and C₂₋₁₂ alkynylene, each of which may contain one or two 5-7-membered rings, which may optionally be condensed; R³ is absent or selected from C₁₋₁₅ alkylene, C₂₋₁₅ alkenylene, and C₂₋₁₅ alkynylene. Each of these groups may be unsubstituted or substituted by 1-3 substituents, wherein said substituents are preferably selected from C₁₋₈ alkyl, C₂₋₈ alkenyl, C₂₋₈ alkynyl, C₁₋₆ alkoxy. Each of R^{a} and R^{b} is independently selected from hydrogen, alkyl groups having 1 to 24 carbon atoms, and glycerol. One or more diacids (a) may be used either singly or in combination.

In a preferred embodiment, R¹ is absent; R² is selected from C₃₋₁₀ alkylene, and C₄₋₁₀ alkenylene, each being unsubstituted or substituted by 1-3 substituents, wherein said substituents are selected from C₁₋₆ alkyl; R³ is absent. Each of R^{a} and R^{b} is independently selected from hydrogen and alkyl groups having 1 to 4 carbon atoms.

In another preferred embodiment diacid (a) is a dimer of a fatty acid, wherein dimerization may for instance involve a 4+2 cycloaddition to yield a 6-membered cycle. Suitable dimers are described for instance in Gubbles et al. Macromolecules (2013), 46, 3975-3984 and Vendamme et al. Macromolecules (2013), 46, 3395-3405.

Fatty acids suitable for dimerization may be selected from mono- and polyunsaturated fatty acids such as oleic acid, linoleic acid, stearidonic acid, eicosatrienoic acid, eicosatetraenoic acid, eicosapentaenoic acid, clupanodonic acid, docosahexaenoic acid, tetracosapentaenoic acid, nisinic acid, mead acid, arachidonic acid, adrenic acid or calendic acid, and/or mixtures thereof as well as palmitoleic acid, eleostearic acid, ricinoleic acid, vernolic acid, licanic acid, myristoleic acid, margaroleic acid, gadoleic acid, eicosadienoic acid and/or erucic acid or mono- or dialkyl esters and mixtures thereof. In a further preferred embodiment, the fatty acid is selected from the group consisting of a linoleic, linolenic, stearidonic, eicosadienoic, eicosatrienoic, eicosatetraenoic, eicosapentaenoic, clupanodonic, docosahexaenoic, tetracosapentaenoic, nisinic, mead, arachidonic, adrenic or calendic acid or mono- or dialkyl esters and mixtures thereof. The dimer may be partially or fully hydrogenated after dimerization.

Dimers of fatty acid are commercially available. For example, dimers of oleic acid or linoleic acid are available from Croda (The Netherlands) under the trade name PRIPOL 1009 or PRIPOL 1006, or Oleon (Belgium) under the trade names Radiacid 0976 or Radiacid 0975. These compounds have high purity, are fully hydrogenated, and are distilled aliphatic dimer acids. These products appear as colorless to lightly colored, clear viscous liquids. Preferred properties can be summarized as follows:

| | |
|---|---|
| *Purity* | *≥95%* |
| *Acid value* | *194-201 mg KOH*/*g* |

Partially hydrogenated dimers of fatty acid can be obtained for instance from Arizona Chemicals (The Netherlands), under the trade mark UNIDYME.

In another preferred embodiment in combination with any of the above or below embodiments, the dimer of fatty acid used for the polycondensation is fully hydrogenated.

### Diol (b):

Diol (b) is selected from the compounds characterized by the following general formula (II):

HO-R⁴-R⁵-R⁶-OH (II)

wherein R⁴ is absent or selected from C₁₋₁₅ alkylene, C₂₋₁₅ alkenylene, and C₂₋₁₅ alkynylene; R⁵ is selected from C₁₋₁₂ alkylene, C₂₋₁₂ alkenylene, and C₂₋₁₂ alkynylene, each of which may contain one or two 5-7-membered rings, which may optionally be condensed; R⁶ is absent or selected from C₁₋₁₅ alkylene, C₂₋₁₅ alkenylene, and C₂₋₁₅ alkynylene. Each of these groups may be unsubstituted or substituted by 1-3 substituents, wherein said substituents are preferably selected from C₁₋₆ alkyl, C₂₋₆ alkenyl, C₂₋₆ alkynyl, C₁₋₆ alkoxy. One or more diols (b) may be used either singly or in combination.

In a preferred embodiment, R⁴ is absent; R⁵ is selected from C₃₋₁₀ alkylene, and C₄₋₁₀ alkenylene, each of which may be unsubstituted or substituted by 1-3 substituents, wherein said substituents are preferably selected from C₁₋₆ alkyl; R⁶ is absent.

In another preferred embodiment, diol (b) is a dimer of a fatty alcohol, wherein dimerization may for instance involve a 4+2 cycloaddition to yield a 6-membered cycle. Another preferred diol (b) is 1,4-butanediol. A combination of both fatty alcohol dimer and 1,4-butanediol is also preferred.

Dimers of fatty alcohols are commercially available, for example, from Croda (The Netherlands) under the trade name PRIPOL 2033. This compound has high purity, is fully hydrogenated, and is a distilled aliphatic dimer alcohol. This product appears as colorless to lightly colored, clear viscous liquid. Preferred properties of this compound can be summarized as follows:

| | |
|---|---|
| *Dimer %* | *≥98%* |
| *Hydroxyl value* | *202-212mg KOH*/*g* |
| *Acid Value* | *≤0.2mg KOH*/*g* |

A partially hydrogenated dimer of fatty alcohol can be obtained for instance from Nanjing HongBaoLi Co., Ltd (China).

In another preferred embodiment in combination with any of the above or below embodiments, the dimer of fatty alcohol used for the polycondensation is fully hydrogenated.

1,4-butanediol is commercially available from BioAmber for example (USA). In a preferred embodiment in combination with any of the above or below embodiments, 1,4-butanediol is present in a concentration of 5 wt% to 40 wt%, more preferably 10 wt% to 20 wt%, relative to the polycondensate.

### Optional further components (c):

The polycondensate (A) may comprise repeating units derived from one or more further components comprising two or more functional groups that are reactive under the polycondensation reaction conditions specified below, provided such optional additional components do not prevent accomplishing the beneficial effects of the present invention, including in particular the beneficial combination of skin friendliness and high.

Optional further components (c) include polyalkylene glycol such as a polyethylene glycol (PEG), poly(tetramethylene ether)glycol (PTMEG), polypropylene glycol (PPG), The present invention, however should not be seen as limited to these specific polymers.

The polyalkylene glycol (c) for optional use in the present invention has a weight average molecular weight of 2000 g/mol or less. It is preferably characterized by the following general formula (III):

H-(O-R⁸)ₙ₁-OH (III)

wherein R⁸ represents an alkylene group having between 2 and 4 carbon atoms, including especially ethylene groups (-CH₂-CH₂-), propylene groups (-CH₂-CH₂-CH₂-), methyl-substituted ethylene (-CH(Me)-CH₂-, wherein Me represents a methyl group), tetramethylene (-CH₂-CH₂-CH₂-CH₂-), methyl-substituted propylene (-CH(Me)-CH₂-CH₂- or -CH₂-CH(Me)-CH₂-), dimethyl-substituted ethylene (-CH(Me)-CH(Me)- or -C(Me)₂-CH₂-) or mixtures thereof, with ethylene groups being preferred (polyethylene glycol, PEG); and
wherein n1 is an average value and it is >1.

Preferably, n1 is in the range of from 2 to 35, more preferably from 3 to 27, even more preferably from 4 to 23 and most preferably from 4 to 15. For the preferred embodiment of PEG, this means that the preferred weight-average molecular weight of component (c) is from 100 to 1500 g/mol, more preferably 150 to 1200 g/mol, even more 180 to 1000 g/mol and most preferably from 190 to 650 g/mol. Particularly preferred is the use of any one of the commercially available products PEG 200, PEG 400, PEG 600, PEG 800, PEG 1000 and PEG 1500 and any mixtures thereof. It is also possible to use different grades of the product, including industrial grade products and highly pure analytical grade products.

When using polyalkylene oxides other than PEG, the average number of repeating units should be the same. Hence, the molecular weight ranges and preferred molecular weight ranges given for PEG may be adapted accordingly, taking the differences in molecular weight of each repeating unit into account.

Further optional components may for instance be compounds containing one or more hydroxyl groups and one or more carboxyl groups. Bifunctional compounds of this type such as lactic acid could serve to replace simultaneously dicarboxylic acid compounds (a) and diol compounds (b). Compounds having more than two functional groups could have the same effect of replacing both components (a) and (b) and, additionally, they could also be used to introduce branching into the polycondensate. Typical compounds of this type are citric acid and citric acid ester.

Branching may also be introduced using optional components (c) that have three or more functional groups of the same type. Typical representatives of this class of optional components (c) are glycerol and pentaerythritol.

Yet further optional components may be higher aggregates, such as trimers or oligomers, that may be formed in the course of the above-mentioned dimerization reactions yielding dimers of fatty acids and dimers of fatty alcohols.

### Compositional ratios:

In a preferred embodiment in combination with any of the above or below embodiments, the optional further component (c) is present in a concentration of 1 mol% to 60 mol% and preferably below 25 mol% relative to the total weight of the feed composition for making the polycondensate. If optional component (c) is a diol, the relative amount of diol (b) is reduced by a corresponding amount. If optional component (c) is a diacid, the relative amount of diacid (a) is reduced by a corresponding amount. If the optional component (c) has both hydroxyl groups and carboxyl groups, the relative amount of both diacid (a) and diol (b) are reduced such that the reduction of the molar amount of carboxyl groups and hydroxyl groups in the two components, respectively, corresponds to the molar amount of carboxyl groups and hydroxyl groups introduced by the optional component (c).

In a preferred embodiment in combination with any of the above or below embodiments, the optional further component (c) is a diol selected from PEG and PTMEG and accounts for 10 mol% to 90 mol%, more preferably 15 mol% to 45 mol%, of the total diol monomers in the feed composition for making the polycondensate.

### 5.2.2 Polycondensation

In a preferred embodiment in combination with any of the above or below embodiments, the molar ratio of component diol (b) (or combination of diols) to component diacid (or combination of diacids) (a) is from 2:1 to 1:2, more preferably from 1.3:1 to 1:1.3. It is even more preferred to use an excess of the diol component such that the molar ratio of diol (b) to diacid (a) is in the range of from (1.05 to 1.15) : 1. An excess of diol component or diacid component results in polycondensate having a lower molecular weight and high hydroxyl or carboxylic acid functional groups respectively.

It is preferred to carry out the polycondensation without solvent, i.e. as a bulk condensation. The stirring speed is preferably in the range of from 120 to 250 rpm, more preferably 150 to 220 rpm. The reaction mixture is preferably heated to a temperature of 160 to 250°C, more preferably 170°C to 230°C. The application of a vacuum in the range of 2 to 60 mbar, preferably 2 to 20 mbar and more preferably 4 to 12 mbar is advantageous. If volatile compounds are used as monomers, the polycondensation is first carried out at normal pressure under a nitrogen stream for 3 to 30 hours, preferably 7 to 20, and then vacuum as specified above is applied. If no volatile monomer compounds are used, said vacuum may be applied even earlier. If no volatile monomer compounds are used, it is particularly preferred to carry out polycondensation in two stages, wherein the first stage involves a polycondensation at a lower vacuum of about 7-13 mbar and a temperature of about 170 to 190°C, and wherein the second stage involves polycondensation at a higher vacuum of about 2-8 mbar and a temperature of about 215-235°C. It is further preferred that the first stage has a duration of 1-3 hours and the second stage has a duration of 2-5 hours.

In a preferred embodiment in combination with any of the above or below embodiments the polycondensation is performed in presence of a catalyst.

A catalyst can be employed for the polycondensation reaction. This catalyst may encompass, but is not limited to: di-ⁿbutyltin (IV) oxide, dioctyltin (IV) dilaurate, titanium (IV) acetyl acetonate, titanium (IV) tetraisopropoxide, titanium(IV) ⁿbutoxide, titanium(IV) ^{t}butoxide, aluminum (III) acetylacetonate, aluminum (III) isopropoxide, iron (III) acetylacetonate, zirconium (IV) acetylacetonate, zirconium (IV) propoxide, tetrakis (acetylacetonato) hafnium (IV), zinc(II) acetylacetonate, molybdenum (IV) oxide bis(acetylacetonate), lantan (III) isopropoxide, vanadium (IV) oxide acetylacetonate.

Non-toxic catalysts such as titanium-based catalysts are preferred over toxic catalysts such as zinc- or tin-based catalysts.

In a more preferred embodiment the polymerization is performed in the presence of Titanium(IV) butoxide as catalyst. More preferably, the catalyst, such as titanium(IV) butoxide, is present at concentration comprised between 0.001 wt% to 1 wt% with respect to the weight of diacid. It is preferred to employ the lowest possible amount of catalyst that yields a satisfactory yield at an acceptable reaction time. Using more catalyst than necessary will lead to darker polycondensates. The minimum required amount of catalyst can be determined by systematic routine optimization procedures.

In a preferred embodiment the polymerization is performed in presence of a catalyst that does not induce darkening or coloring of the polycondensate.

### 5.2.3 Molecular Weight and Molecular Weight distribution

The polycondensate (A) is typically characterized by a molecular weight of from 20,000 to 85,000 g/mol, preferably from 27,000 to 70,000 g/mol and even more preferably from 50,000 to 65,000 g/mol. It is furthermore preferable that the polycondensate (A) exhibits a molecular weight distribution Mw/Mn of from 1.00 to 3.5, preferably from 1.4 to 2.5 and even more preferably from 1.5 to 2.0.

The adhesive level is influenced by the polymer molecular weight, and a molecular weight ≥ 50 000 g/mol is preferred in order to get good adhesion on human skin. Furthermore the high molecular weight is an essential parameter of the device since it allows the incorporation of a high amount of oil with the adhesive structure.

### 5.2.4 Further Properties

The polycondensate (A) is preferably characterized by one or more of the following further properties:
- α transition temperature below 0°C, preferably from -80°C to -20°C., even more preferably from -60°C to -50°C.
- acid value (expressed in mg KOH/g polymer) from 0.1 to 150, preferably from 0.1 to 20, even more preferably from 0.1 to 1.
- hydroxyl value (expressed in mg KOH/g polymer) from 1 to 150, preferably from 1 to 50, even more preferably from 15 to 25.

The acid value is defined as the mass of potassium hydroxide (in mg) required to neutralized 1g of polycondensate, and is measured by direct titration using a standard ethanolic potassium hydroxide solution.

The hydroxyl value is defined as the mass of potassium hydroxide (in mg) equivalent to the hydroxyl content of 1g of polycondensate. It is measured by acetylation of the polycondensate followed by hydrolysation of the excess of acetic anhydride. The acetic acid formed is subsequently titrated with an ethanolic potassium hydroxide solution.

### 5.3 Adhesive and adhesive preparation

### 5.3.1 Overview

The adhesive of the present invention is a composition comprising the above polycondensate (A) in a cross-linked form together with an oil additive as essential components. Further optional components may be present as long as these do not prevent accomplishing the beneficial effects of the present invention.

### 5.3.2 Cross-linking agent

Crosslinking of the above polycondensate (A) is accomplished by reacting the polycondensate with one or more cross-linking agents.

In a preferred embodiment in combination with any of the above or below embodiments, the cross-linking agent has at least 2 functional groups selected from the group consisting of an isocyanate, an epoxide, and an anhydride, and any combination thereof. In one embodiment, the cross-linking agent is different from the epoxidized fatty acid esters employed in WO 2011/023255 A.

In a preferred embodiment in combination with any of the above or below embodiments, a cross-linking agent having a structure R⁷-(N=C=O)ₙ is employed, wherein R⁷ is an C₁₋₅₀aliphatic, C₄₋₅₀alicyclic or C₆₋₅₀aromatic residue, and n is an integer ≥2.

In a preferred embodiment in combination with any of the above or below embodiments, R⁷ is an aliphatic, alicyclic or aromatic residue containing from 10 to 40 carbon atoms. In a preferred embodiment in combination with any of the above or below embodiments, n is an integer of 2, 3, 4, 5, or 6, more preferably of 2, 3 or 4, in particular 3.

A typical example of a commercially available aliphatic polyisocyanate crosslinking agent is produced by Bayer (Germany), under the trade mark DESMODUR or Japan Polyurethane under the Trade mark CORONATE.

In one embodiment, a mixture of two or more crosslinking agents is used, wherein n2 in one crosslinking agent is 2 and n2 in one or more other crosslinking agents is 3 or more. The crosslinking agent with n2 being 2 is preferably a compound selected from the group consisting of C₅-C₁₂ alkanes, more preferably C₆ alkanes, carrying two isocyanate groups. The alkane can be linear, branched or cyclic with linear alkanes being more preferred. The two isocyanate groups can be attached to any carbon atom of the alkane. Attachment of the isocyanate groups to two opposite terminal carbon atoms is more preferred. A particularly preferred crosslinker is 1,6-diisocyanatohexane.

According to another preferred embodiment, a cross-linking agent may be used, which is a linear aliphatic NCO prepolymer based on C₃₋₁₀ alkylene diisocyanate, preferably hexamethylene diisocyanate. A commercially available product of this type is Baymedix^{®} AP501 from BAYER.

### 5.3.3 Cross-linking reaction

In a preferred embodiment the crosslinking agent is present at concentration of 0.1 wt% to 20 wt% more preferably 2 wt% to 10 wt%, relative to the polycondensate (A) prior to cross-linking. If an linear aliphatic NCO prepolymer is used, the amount should preferably be in the range 10-20 wt%.

It is possible to carry out the cross-linking reaction by heat or using a catalyst or a combination of heat and catalyst.

In a preferred embodiment the crosslinking agent, polycondensate, oil additive and optional component are mixed together for the crosslinking reaction.

In another preferred embodiment the crosslinking agent, polycondensate, oil additive, optional component and an organic solvent are mixed together for the crosslinking reaction. The organic solvent employed can be aromatic (such as toluene) or non-aromatic (such as ethyl acetate). The concentration of the organic solvent employed is comprised from 0.1 to 100 weight %, preferably from 5 to 50 weight %, even more preferably from 10 to 30 weight %, (with regard to the polycondensate).

Crosslinking reaction temperature can be selected from 25°C to 200°C, preferably from 100°C to 180°C, even more preferably from 120°C to 150°C.

Crosslinking reaction time can be selected from 1 to 600 minutes, preferably from 1 to 20 minutes, even more preferably from 3 to 8 minutes.

In one embodiment, the crosslinking reaction is carried out without use of a polymerization catalyst as specified in WO 2011/023255 and especially without dibutyltin oxide, titanium tetrabutoxide, 1,5,7-triazabicyclo[4.4.0]dec-5-ene (TBD), 1,5-diazabicyclo[4.3.0]non-5-ene (DBN) and 1,8-diazabicyclo[5,4.0]undec-7-ene (DBU).- In another embodiment, especially in large scale production, the use of a catalyst is contemplated. Catalysts that can be employed for the crosslinking reaction encompass, but are not limited to: di-ⁿbutyltin (IV) oxide, dioctyltin (IV) dilaurate, titanium (IV) acetyl acetonate, titanium (IV) tetraisopropoxide, titanium(IV) ⁿbutoxide, titanium(IV) ^{t}butoxide, aluminum (III) acetylacetonate, aluminum (III) isopropoxide, iron (III) acetylacetonate, zirconium (IV) acetylacetonate, zirconium (IV) propoxide, tetrakis (acetylacetonato) hafnium (IV), zinc(II) acetylacetonate, molybdenum (IV) oxide bis(acetylacetonate), lantan (III) isopropoxide,vanadium (IV) oxide acetylacetonate.

Non-toxic catalysts such as titanium-based catalysts are preferred over toxic catalysts such as zinc- or tin-based catalysts.

In a more preferred embodiment the curing is performed in the presence of titanium(IV) butoxide as catalyst. More preferably, the catalyst, such as titanium(IV) butoxide, is present at concentration comprised between 0.001 wt% to 10 wt% with respect to the weight of the polycondensate. It is preferred to employ the lowest possible amount of catalyst that yields a satisfactory yield at an acceptable reaction time. Using more catalyst than necessary will lead to darker polycondensates. The minimum required amount of catalyst can be determined by systematic routine optimization procedures.

In a preferred embodiment the curing is performed in presence of a catalyst that does not induce darkening or coloring of the polycondensate.

### 5.3.4 Cross-linked polycondensate

In a preferred embodiment in combination with any of the above or below embodiments, the adhesive gel content value of the cross-linked polycondensate is comprised between 60 % to 95%, most preferably between 70 % to 80%.

### 5.3.5 Oil additive (B)

The adhesive of the present invention contains an oil additive (B). Said oil additive may be selected from any natural or synthetic, organic or mineral oil which is suitable for topical application to the human skin. The oil additive may also be selected from the group of softeners and plasticizers employed in polymer industry, provided the softener or plasticizer is suitable for topical application to the human skin. In a preferred embodiment the oil additive is colorless, and its viscosity is lower than that of the polycondensate. It is also preferred that the polycondensate is fully soluble in the oil additive or in the mixture of the oil additive and an organic solvent.

In a preferred embodiment in combination with any of the above or below embodiments, the adhesive contains the oil additive at a concentration from 1 wt% to 150 wt% more preferably 80 wt% to 120 wt%, relative to the polycondensate.

In another preferred embodiment in combination with any of the above or below embodiments, the oil additive is bio-based.

For ensuring good cohesion of the final product, it is important to ensure that the adhesive composition, including polycondensate and oil (if present), forms a homogeneous solution or mixture both before and after curing. Hence, if a particular composition should not be homogeneous, it is preferred to reduce the amount of oil present and/or to replace the oil component by another oil component that has a higher degree of hydrophilicity in order to ensure that the composition is homogeneous.

In another preferred embodiment in combination with any of the above or below embodiments, the oil additive does not contain any free carboxylic acid (either as part of the tackifier or as an impurity) in order to reduce risk of skin irritation.

In another preferred embodiment in combination with any of the above or below embodiments, the oil additive does not contain unsaturation. The absence of unsaturated groups is helpful in order to avoid undesired side reactions, and "yellowing" effects if the adhesive is sterilized. Aromatic ring-containing chemical structures are also not preferable.

The oil additive can for instance be one or more oils selected from the following list, but it is not limited thereto: sorbitan mono- or di-esters of fatty acids such as sorbitan ester of oleic acid, oleate esters, soybean oil, citrate ester, vernonia oil, PEG (Mw 100-1000) and PEG derivatives such as PEG ether (such as dimethyl PEG) or PEG esters such as PEG distearate, fatty acids such as myristic acid, palmitic acid, stearic acid, lauric acid, oleic acid, isostearic acid, neodecanoic acid, trimethylhexanoic acid and neoheptanoic acid; the esters of these fatty acids such as isopropyl myristate; fatty alcohols such as myristyl alcohol, cetyl alcohol, oleyl alcohol and lauryl alcohol; aliphatic pyrrolidones such as N-lauryl-2-pyrrolidone; terpenes such as 1-menthol, d-limonene and α-terpineol; alkanes such as heptane, octane, nonane and decane; and substances acting as solubilizers and transdermal absorption promoters of medicinal ingredients such as crotamiton and α-, β- and γ-cyclodextrin. Besides, mention may be made of substances for use as fragrant/refreshing materials such as L-menthol, camphor, thymol, mint oil, castor oil, fennel oil, star anise oil, cinnamon oil, oil of cloves, thiamine oil, turpentine oil, eucalyptus oil, lavender oil, lemon oil, orange oil, bergamot oil and rose oil. Additionally, fatty acid esters of monovalent alcohols such as cetyl octanoate, hexyl laurate, isopropyl myristate, isopropyl palmitate, butyl stearate, myristyl lactate, and the like; dibasic acid esters such as dioctyl adipate, diethyl sebacate, dioctyl sebacate, dioctyl succinate, and the like; fatty acid esters of polyvalent alcohols and the like such as propylene glycol dicaprate, glycerol trioctanoate, glycerol tri(octanoate/decanoate), medium chain fatty acid triglyceride, and the like; and in particular, isopropyl myristate, isopropyl palmitate, diethyl sebacate, middle chain fatty acid triglyceride, and the like are preferably used. Further examples of the oil additive include glycols such as ethylene glycol, diethylene glycol, propylene glycol, dipropylene glycol, tripropylene glycol, triethylene glycol, poly(propylene glycol) and the like; fats and oils such as olive oil, castor oil and the like; lanolin; hydrocarbons such as squalane and liquid paraffin; various surfactants; ethoxylated stearyl alcohol; glycerol monoesters such as oleic acid monoglyceride, caprylic acid monoglyceride and lauryl acid monoglyceride; dialkyl ester of polyalkylene glycol such as poly(propylene glycol); glycerol diester such as glycerol diacetate and the like, glycerol triester such as glycerol triacetate and the like, or a mixture thereof; fatty acid alkyl ester such as triethyl citrate and the like; long chain alcohol; higher fatty acid such as oleic acid and caprylic acid; alkyl ester of higher fatty acid such as isopropyl myristate; pyrrolidones such as N-methylpyrrolidone and N-dodecylpyrrolidone; sulfoxides such as decyl methyl sulfoxide; 1,3-butanediol and the like. All of the above oil additives can be used alone or in a mixture of two or more kinds thereof.

In another preferred embodiment in combination with any of the above or below embodiments, the oil additive is an isosorbide ester (monoester or diester) or a mixture of two or more isosorbide esters, each of which being selected from isosorbide monoesters or isosorbide diesters. The isosorbide diester employed can be both symmetrical or asymmetrical. Preferably, isosorbide is esterified with one or two fatty acids, both being independently selected from C₆₋₁₈ saturated or unsaturated fatty acids, more preferably C₈₋₁₂ saturated or unsaturated fatty acids. Isosorbide dicaprylate is commercially available from Sytheon (USA) under the trade mark Synovea^{®} DOI. A mixture of isosorbide diesters is commercially available under the trade mark POLYSORB ID 37 (Roquette Frères, France). As an alternative to esters of isosorbide, it is also possible to employ the esters of stereoisomers of isosorbide, namely esters of isomannide or isoidide. However, it is more preferably to employ isosorbide due to its commercial availability and low cost.

Among the above-mentioned oil components, it is particularly preferred to employ one or more oil components selected from the group of sorbitan mono- or di-esters of fatty acids, PEG diesters, sesame oil, POLYSORB ID37, sorbitol mono- and diesters as well as vernonia oil. As a preferred embodiment, an oil is used which has no terminal hydroxyl groups and, more preferably, the oil has no hydroxyl groups at all.

According to one embodiment of the present invention, the adhesive composition is not an adhesive composition comprising a cross-linked polyester and a polyether-polyol, wherein the weight average molecular weight of the polyester before cross-linking is from 5,000 g/mol to 50,000 g/mol, wherein the hydroxyl groups are partially modified or non-modified, wherein when the hydroxy groups are partially modified, the polyether-polyol has the number average molecular weight of from 100 to 1500 (Mn) and the content of said polyether-polyol is 1-35 wt% relative to the polyester, and
wherein when the hydroxy groups are non-modified, the polyether-polyol has the number average molecular weight of from 100 to 5000 (Mn) and especially when the content of said polyether-polyol is 1-400 wt% relative to the polyester.

### 5.3.6 Tackifier

In a preferred embodiment in combination with any of the above or below embodiments, the adhesive contains a tackifier agent or tack enhancer agent or a combination of two or more of such agents (hereinafter jointly referred to as "tackifier"). If present, the tackifier is preferably present at a concentration from 0.1 wt% to 30 wt% more preferably 1 wt% to 5 wt%, relative to the weight of the polycondensate.

In another preferred embodiment in combination with any of the above or below embodiments, the tackifier agent is bio-based.

The tackifier can for instance be rosin or a rosin derivative, but it is not limited to this type of substances. The tackifier is preferentially selected from the group consisting of rosins and their derivates (abietic acid, abietic acid esters), terpenes and their derivatives (polyterpene resins, terpene-phenol resins), aliphatic, cycloaliphatic and aromatic resins (C5 aliphatic resins, C9 aromatic resins, and C5/C9 aliphatic/aromatic resins), hydrogenated hydrocarbon resins, and their mixtures.

Suitable rosin derivatives are for instance:
- Esterified hydrogenated rosin such as: FORALYN 90, FORALYN 110, FORALYN 5020-F (Eastman).
- Ester of tall oil rosin such as: SYLVALITE RE 105XL (Arizona Chemical, The Netherlands).

In another preferred embodiment in combination with any of the above or below embodiments, the tackifier does not contain free carboxylic acid (either as part of the tackifier or as an impurity) in order to reduce risk of skin irritation. In another preferred embodiment in combination with any of the above or below embodiments, the tackifier does not contain unsaturation. The absence of unsaturated groups is advantageous in order to avoid undesired side reactions, and "yellowing" effect if the adhesive is sterilized. Tackifiers containing aromatic rings are also not preferable.

### 5.3.7 Optional further adhesive components

Further components may optionally be present, provided these optional further components do not interfere with the beneficial effects of the invention. Additional components suitable for use in the adhesive of the present invention include the following: moisture regulating agents such as alginates; aginate derivatives and alginate salts (as for instance zinc alginate or silver alginate); starch; cellulose derivatives such as carboxymethyl cellulose (CMC), hydroxyethyl cellulose or acetate cellulose and their salts; gelatin and the like such as agar-agar or pectin; gums (such xanthan gum, gum arabic, guar gum, locust bean gum); collagen; polysaccharide such as chitosan, hyaluronic acid or hyaluronate derivatives and salts; activated charcoal; as well as components having antibacterial activity such as silver particles/crystal and silver salts such as silver sulfadiazine; sulfated molecules and sulfate rich polymers and iodine derivatives.

### 5.3.8 Manufacture of the adhesive of the invention

The adhesive of the invention may be prepared in the following manner: The method for manufacturing the adhesive of the invention is not particularly limited. In a preferred embodiment of practicing the invention, the polymer (polycondensate) is mixed with the oil additive, a solvent or a mixture of solvents (optional), the tackifier (optional) and the crosslinker (crosslinking agent). If the tackifier is a solid, a solution of tackifier in a compatible solvent is preferably used. Once the mixture is homogeneous, the solution is casted on a suitable surface such as a liner and cured. After curing, the "free" side of the adhesive may be laminated onto a semi-finished product, such as a backing, support, protective film or other product, to thereby yield the final product of interest. Alternatively, the "free" side of the adhesive may be simply laminated onto a further liner surface for storage purposes.

It may furthermore be advantageous to mix the polymer initially with oil to decrease the viscosity before adding further ingredients. It is also preferable that the crosslinker is added just before coating and curing. The tackifier may be added together with the oil or after the oil addition.

### 5.4. Medical Tape and Patch

### 5.4.1 Backing

To prepare a medical tape or patch, the adhesive is laminated on a backing. Suitable backing materials may, for instance, be selected from woven and non-woven materials, wherein preferred woven backing materials are selected from cotton, nylon, rayon or a mixture thereof; and wherein preferred non-woven materials are preferably selected from polyethylene terephthalate (PET), polyethylene (PE), polyurethane (PU), polyvinyl chloride (PVC), polypropylene (PP), wood pulp, cellulose, starch and/or polylactic acid (PLA).

In a preferred embodiment in combination with any of the above or below embodiments the adhesive is laminated onto a non-woven having one or more of the following properties: weight: 15 - 75 g/m², thickness: 0.10 - 0.5 mm, maximum tensile strength: 70 - 90N/50mm (1.4 - 1.8 N/mm) and maximum elongation 15-45%. It is more preferred to use a backing material fulfilling two of these properties, even more preferably three of these properties and most preferably all four of these properties.

In a preferred embodiment the backing material used can withstand sterilization process without visible changes (e.g. no yellowing).

### 5.4.2 Patch-related Features

The patch of the present invention has a construction and layer structure corresponding to that of a conventional patch. It is characterized by the presence of the adhesive of the present invention instead of the presence of a standard pressure-sensitive adhesive.

Hence, the patch of the present invention may comprise one or more of the following features: structure consisting of non-adhesive area and adhesive area; structure comprising non-adhesive fluid absorbent material and adhesive area; structure comprising moisture regulation material/compound and adhesive area; structure containing active agent reservoir material and adhesive area.

The present invention also encompasses patches, wherein adhesive thickness is not the same everywhere in the patch. This can be, for instance, patches, wherein the adhesive is thicker or thinned at the edge.

The present invention also encompasses patches that contain an active pharmaceutical ingredient, which is to be administered to the patient by transdermal or topical delivery.

The active pharmaceutical ingredient to be delivered is not particularly limited. It is for instance possible to incorporate an active pharmaceutical ingredient selected from one of the following classes of drugs (it will be appreciated that some of biologically active compounds can be classified in more than one of these classes):
Cardiovascular drugs, in particular antihypertensive agents (e.g. calcium channel blockers or calcium antagonists) and antiarrhythmic agents; congestive heart-failure pharmaceuticals; inotropic agents; vasodilators; ACE inhibitors; diuretics; carbonic anhydrase inhibitors; cardiac glycosides; phosphodiesterase inhibitors; a blockers; [beta]-blockers; sodium channel blockers; potassium channel blockers; [beta]-adrenergic agonists; platelet inhibitors; angiotensin II antagonists; anticoagulants; thrombolytic agents; treatments for bleeding; treatments for anaemia; thrombin inhibitors; antiparasitic agents; antibacterial agents; insulin; human growth hormone and peptides; vaccines; antiinflammatory agents, in particular non-steroidal antiinflammatory agents (NSAIDs), more particularly COX-2 inhibitors; steroidal antiinflammatory agents; prophylactic antiinflammatory agents; antiglaucoma agents; mast cell stabilisers; mydriatics; agents affecting the respiratory system; allergic rhinitis pharmaceuticals; alpha-adrenergic agonists; corticosteroids; chronic obstructive pulmonary disease pharmaceuticals;xanthine-oxidase inhibitors; antiarthritis agents; gout treatments; autacoids and autacoid antagonists; antimycobacterial agents; antifungal agents; antiprotozoal agents; anthelmintic agents; antiviral agents especially for respiratory , herpes, cytomegalovirus, human immunodeficiency virus and hepatitis infections; treatments for leukemia and kaposi's sarcoma; pain management agents in particular opioids, anaesthetics and analgesics; neuroleptics; sympathomimetic pharmaceuticals; adrenergic agonists; drugs affecting neurotransmitter uptake or release; anticholinergic pharmaceuticals; antihaemorrhoid treatments; agents to prevent or treat radiation or chemotherapeutic effects; liopgenisis drugs; fat reducing treatments; anti-obesity peptides; antiobesity agents such as lipase inhibitors; sympathomimetic agents; treatments for gastric ulcers and inflammation such as proton pump inhibitors; prostaglandins; VEGF inhibitors; antihyperlipidemic agents, in particular statins; drugs that affect the central nervous system (CNS) such as antipsychotic, antiepileptic and antiseizure drugs (anticonvulsants), psychoactive drugs, stimulants, antianxiety and hypnotic drugs, antidepressant drugs; anti-Parkinson's pharmaceuticals; hormones and fragments thereof such as sex hormones; growth hormone antagonists; gonadotropin releasing hormones and analogues thereof; steroid hormones and their antagonists; selective estrogen modulators; growth factors; antidiabetic pharmaceuticals such as insulin, insulin fragments, insulin analogues, glucagon-like peptides and hypoglycaemic agents; H1, H2, H3 and H4 antihistamines; peptide, protein, polypeptide, nucleic acids and oligonucleotide pharmaceuticals; analogues, fragments and varients of natural proteins, polypeptides, oligonucleaotides and nucleic acids and such like compounds; agents used to treat migraine headaches; asthma pharmaceuticals; cholinergic antagonists; glucocorticoids; androgens; antiandrogens; inhibitors of adrenocorticoid biosynthesis; osteoporosis treatments such as biphosphonates; antithyroid pharmaceuticals; suncreens, sun protectants and filters; cytokine agonists; cytokine antagonists; anticancer drugs; anti-Alzheimer drugs; HMGCoA reductase inhibitors; fibrates; cholesterol absorption inhibitors; HDL cholesterol elevating agents; triglyceride reducing agents; antiageing or antiwrinkle agents; precursor molcules for the generation of hormones; proteins such as collagen and elastin; antibacterial agents; anti acne agents; antioxidants; hair treatments and skin whitening agents; suncreens, sun protectants and filters; variants of human apolipoprotein; precursor molecules for generation of hormones; proteins and peptides thereof; amino acids; plant extracts such as grape seed extract; DHEA; isoflavones; nutritional agents including vitamins, phytosterols and iridoid gylcosides, sesquiterpene lactones, terpenes, phenolic glycosides, triterpenes, hydroquinone derivatives, phenylalkanones; antioxidants such as retinol and other retinoids including retinoic acid and co-enzyme Q10; omega-3-fatty acids; glucosamine; nucleic acids, oligonucleotides, antisense pharmaceuticals; enzymes; cytokines; cytokine analogues; cytokine agonists; cytokine antagonists; immunoglobulins; antibodies; antibody pharmaceuticals; gene therapies; lipoproteins; erythropoietin; vaccines; small and large molecule therapeutic agents for the treatment, or prevention of human and animal diseases such as allergy/asthma, arthritis, cancer, diabetes, growth impairment, cardiovascular diseases, inflammation, immunological disorders, baldness, pain, ophthalmological diseases, epilepsy, gynaecological disorders, CNS diseases, viral infections, bacterial infections, parasitic infections, GI diseases, obesity, and haemological diseases. Some specific non-limiting examples of suitable biologically active compounds include: anaesthetics: including amino-ester and amino-amide anaesthetics such as benzocaine, chloroprocaine, cocaine, reserpine, guanethidine, cyclomethycaine, dimethocaine/larocaine, propoxycaine, procaine/novocaine, proparacaine, tetracaine/amethocaine; articaine, bupivacaine, carticaine, cinchocaine/dibucaine, etidocaine, levobupivacaine, lidocaine/lignocaine, mepivacaine, piperocaine, prilocaine, ropivacaine, trimecaine, propofol, halothane, enflurane barbiturates, benzodiazepines, neostigmine and ketamine alkylating agents: including carmustine, cyclophosphamide, ifosfamide, streptozotocin and mechlorethamine calcium channel blockers: including amlodipine, aranidipine, azelnidipine, barnidipine, benidipine, cilnidipine, clevidipine, cronidipine, darodipine, dexniguldipine, efonidipine, elnadipine, elgodipine, felodipine, flordipine, furnidipine, iganidipine, isradipine, lacidipine, lemildipine, lercanidipine, manidipine, mesuldipine, nicardipine, nifedipine, niludipine, nilvadipine, nimodipine, nisoldipine, nitrendipine, olradipine, oxodipine, palonidipine, pranidipine, sagandipine, sornidipine, teludipine, tiamdipine, trombodipine, watanidipine, verapamil, gallopamil, benzothiazepine, diltiazem, mibefradil, bepridil, fluspirilene and fendiline antiarrhythmic and antiangina agents: including amiodarone, disopyramide, flecainide acetate, quinidine sulphate, nitroglycerine, ranolazine, amiodarone, isosorbide and alteplase antibacterial, antibiotic and antiacne agents: including amoxicillin, ampicillin, azithromycin, benethamine penicillin, bleomycin, benzoyl peroxide, cinoxacin, chloramphenicol, daunorubicin, plicamycin, fluoroquinolones, ciprofloxacin, clarithromycin, clindamycin, clindesse, clofazimine, chlorohexidine gluconate, cloxacillin, demeclocycline, doxycycline, erythromycin, ethionamide, imipenem, indomethacin, lymocycline, minocycline, nalidixic acid, nitrofurantoin, penicillin, rifampicin, spiramycin, sodium sulfacetamide, sulphabenzamide, sulphadoxine, sulphamerazine, sulphacetamide, sulphadiazine, sulphafurazole, sulphamethoxazole, sulphapyridine, tetracycline, cephalexin, cefdinir, triclosan, ofloxacin, vancocin, glyburide, mupirocin, cefprozil, cefuroxime axetil, norfloxacin, isoniazid, lupulone, D-penicillamine, levoffoxacin, gatifoxacin, and trimethoprim anticancer: including doxorubicin, 6-thioguanine, paclitaxel, docetaxel, camptothecin, megestrol acetate, navelbine, cytarabine, fludarabine, 6-mercaptopurine, 5-fluorouracil, teniposide, vinblastine, vincristine, cisplatin, colchicine, carboplatin, procarbazine and etopside antidepressants, antipsychotics and antianxiety: including alprazolam, amoxapine, bentazepam, bromazepam , clorazipine, clobazam, clotiazepam, diazepam, lorazepam, flunitrazepam, flurazepam, lormetazepam, medazepam, nitrazepam, oxazepam, temazepam, maprotiline, mianserin, nortriptyline, risperidone, sertraline, trazodone, baloperidol, trimipramine maleate fluoxetine, ondansetron, midazolam, chlorpromazine, haloperidol, triazolam, clozapine, fluopromazine, fluphenazine decanoate, fluanisone, perphenazine, pimozide, prochlorperazine, sulpiride, thioridazine, paroxitine, citalopram, bupropion, phenelzine, olanzapine, divalproex sodium and venlafaxine tricyclics: including azothiopine, amitriptyline, famotidine, promethazine, paroxatine, oxcarbazapine and mertazapine antidiabetics: including acetohexamide, chlorpropamide, glibenclaraide, gliclazide, glipizide, metformin, tolazamide, glyburide, glimepiride and tolbutamide antiepileptics: including beclamide, carbamazepine, gapapentin, tiagabine, vigabatrin, topiramate, clonazepam, ethotoin, methoin, methsuximide, methylphenobarbitone, oxcarbazepine, paramethadione, phenacemide, phenobarbitone, phenyloin, phensuximide, primidone, sulthiamine, phenytoin sodium , nirofurantoin monohydrate, gabapentin, lamotrigine, zonisamide, ethosuximide and valproic acid hypnotics/sedatives and muscle relaxants: including Zolpidem tartrate, amylobarbitone, barbitone, butobarbitone, pentobarbitone, brotizolam, carbromal, chlordiazepoxide, chlormethiazole, ethinamate, meprobamate, methaqualome, cyclobenzaprene, cyclobenzaprine, tizanidine, baclofen, butalbital, zopiclone, atracurium, tubocurarine and phenobarbital antifungal, antiprotazoal and antiparasitic agents: including amphotericin, butoconazole nitrate, clotrimazole, econazole nitrate, fluconazole, flucytosine, griseofulvin, itraconazole, ketoconazole, miconazole, natamycin, nystatin, sulconazole nitrate, terconazole, tioconazole and undecenoic acid; benznidazole, clioquinol, decoquinate, diiodohydroxyquinoline, diloxanide furoate, dinitolmide, furzolidone, metronidazole, nimorazole, nitrofurazone, ornidazole, terbinafine, clotrimazole, chloroquine, mefloquine, itraconazole, pyrimethamine, praziquantel, quinacrine, mebendazole and tinidazoleantihypertensive and cardiac therapeutic agents: including candesartan, hydralazine, clonidine, triamterene, felodipine, gemfibrozil, fenofibrate, nifedical, prazosin, mecamylamine, doxazosin, dobutamine and cilexetil anti migraine agents: including dihydroergotamine mesylate, ergotamine tartrate, methysergide maleate, pizotifen maleate and sumatriptan succinate antimuscarinic agents: including atropine, benzhexol, biperiden, ethopropazine, hyoscyamine, mepenzolate bromide, oxybutynin, oxyphencylcimine and tropicamide antineoplastic agents (or immunosuppressants): including aminoglutethimide, amsacrine, azathioprine, busulphan, chlorambucil, cyclosporin, dacarbazine, estramustine, etoposide, lomustine, melphalan, mercaptopurine, methotrexate, mitomycin, mitotane, mitozantrone, procarbazine, tamoxifen citrate, testolactone, tacrolimus, mercaptopurine and sirolimus anti-Parkinsonian agents: including bromocriptine mesylate, levodopa, tolcapone, ropinirole, bromocriptine, hypoglycaemic agents such as sulfonylureas, biguanides, α-glucosidase inhibitors, thaiazolidinediones, cabergoline, carbidopa and lysuride maleate antithyroid agents: including carbimazole and propylthiouracil antiviral drugs: including amantadine, retinovir, cidofovir, acyclovir, famciclovir, ribavirin, amprenavir, indinavirm, rimantadine and efavirenz, penciclovir, ganciclovir, vidarabine, abacavir, adefovir, apmrenavir, delavirdine, didanosine, stavudine, zaicitabine, zidovudine, enfuvirtide and interferon cardiac inotropic agents: including amrinone, milrinone, digitoxin, digoxin, enoximone, lanatoside C and medigoxin hypo and hyper lipidemic agents: including fenofibrate, clofibrate, probucol, ezetimibe and torcetrapib antiinflammatory: including meoxicam, triamcinolone, cromolyn, nedocromil, hydroxychloroquine, montelukast, zileuton, zafirlukast and meloxicam antihistamine: including fexofenadine, chloral hydrate, hydroxyzine, promethazine, cetirazine, cimetidine, clyclizine, meclizine, dimenhydrinate, loratadine, nizatadine and promethazine antiulcer: including omeprazole, lansoprazole, pantoprazole and ranitidine diuretics: including hydrochlorothiazide, amiloride, acetazolamide, furosemide and torsemide opioids: including natural opiates which are alkaloids contained in the resin of the opium poppy such as morphine, codeine and thebaine; semi-synthetic opioids created from natural opiates such as hydromorphone, hydrocodone, oxycodone, oxymorphone, desomorphine, diacetylmorphine (heroin), nicomorphine, dipropanoylmorphine, benzylmorphine and ethylmorphine; fully synthetic opioids such as fentanyl, pethidine, methadone, tramadol and dextropropoxyphene; and, endogenous opioid peptides, produced naturally in the body, such as endorphins, enkephalins, dynorphins, and endomorphins; opioid analgesics including opioid receptor agonists, opioid receptor partial agonists, opioid antagonist or opioid receptor mixed agonist-antagonists; opioid receptor agonists including morphine, depomorphine, etorphine, heroin, hydromorphone, oxymorphone, levorphanol, methadone, levomethadyl, meperidine, fentanyl, sufentanyl, alfentanil, codeine, hydrocodone, oxycodone, and mixtures of the foregoing; opioid receptor antagonists including naloxone and naltrexone; opioid receptor mixed agonist-antagonist which has mixed opioid agonist/antagonist activities, or one that exhibits only partial agonist activity, including buprenorphine, nalbuphine, butorphanol, pentazocine, and mixtures of such compounds; opioids which exhibit partial agonist activity, including ethylketocyclazocine; opium alkaloids including phenanthrenes which are naturally occurring in opium such as codeine, morphine, thebaine and oripavine (the active metabolite of thebaine); synthetic derivatives such as diacetylmorphine (heroin), dihydrocodeine, hydrocodone, hydromorphone, nicomorphine, desmorphine, ethylmorphine, dipropanoylmorphine, oxycodone and oxymorphone; synthetic opioids including anilidopiperidines such as fentanyl , alphamethylfentanyl, alfentanil, sufentanil, remifentanil, carfentanyl and ohmefentanyl, phenylpiperidines such as pethidine (meperidine), ketobemidone, MPPP, allylprodine, prodine and PEPAP; diphenylpropylamine derivatives such as propoxyphene, dextropropoxyphene, dextromoramide, bezitramide, piritramide, methadone, dipipanone, levomethadyl acetate (LAAM), difenoxin, diphenoxylate and loperamide; benzomorphan derivatives such as dezocine, pentazocine and phenazocine; oripavine derivatives such as buprenorphine, dihydroetorphine and etorphine; morphinan derivatives such as butorphanol, nalbuphine, levorphanol and levomethorphan, and others such as lefetamine, meptazinol, tilidine, tramadol and tapentadol; opioid receptor antagonists including nalmefene, naloxone and naltrexone NSAIDs: including arylalkanoic acid sub-group of class which includes diclofenac, aceclofenac, acemetacin, alclofenac, bromfenac, etodolac, indometacin, indometacin farnesil, nabumetone, oxametacin, proglumetacin, sulindac and tolmetin; 2-arylpropionic acid (profens) sub-group of class which includes alminoprofen, benoxaprofen, carprofen, dexibuprofen, dexketoprofen, fenbufen, fenoprofen, flunoxaprofen, flurbiprofen, ibuprofen, ibuproxam, indoprofen, ketoprofen, ketorolac, loxoprofen, miroprofen, naproxen, oxaprozin, pirprofen, suprofen, tarenflurbil and tiaprofenic acid; and /V-arylanthranilic acid (fenamic acid) sub-group of class which includes flufenamic acid, meclofenamic acid, mefenamic acid and tolfenamic acid; tromethamine, celecoxib, nepafenac, aspirin, rofecoxib, naproxen, sulindac, piroxicam, pheylbutazone, tolmetin, indomethacin, acetominophen (paracetamol), tramadol and propoxyphene retinoids: including first generation retinoids such as retinol, retinal, tretinoin (retinoic acid, Retin-A), isotretinoin and alitretinoin; second generation retinoids such as etretinate and its metabolite acitretin; third generation retinoids such as tazarotene, bexarotene and adapalene hormones and steroids: including adrenocorticotrophic hormone (ACTH), antidiruetic hormone (vasopressin), atrial- nartreuretic factor (ANF), atrial-nartreuretic peptide (ANP), bedomethasone, cortisone, scopolamine, dopamine, epinephrine, catecholamines, cholecystokinin, clomiphene citrate, danazol, dexamethasone, diethylstilbestrol (DES), ethinyl estradiol, fludrocortison, finasteride, follicle stimulating hormone, gastrin, hydroxyprogesterone, growth hormone, insulin, leptin, luteinizing hormone, medroxyprogesterone acetate, mestranol, quinestrol, methyltestosterone, nandrolone, norethindrone, norethisterone, norgestrel, estradiol, conjugated oestrogens, oxandrolone, oxytocin, prednisone, progesterone, prolactin, protogalndins, somatostatin, stanozolol, stibestrol, thyroxine, prednisolone phosphate, triamcinolone, mifepristone acetonide, budesonide, levothyroxine, testosterone, testosterone cypionate, fluoxymesterone, flutamide, mometasone furoate, cyproterone, fluromethalone, goserelin, leuprolide, calcitonin, halobetasol, hydrocortisol and tibolone statins and derivatives: including atorvastatin, fluvastatin, lovastatin, nystatin, rosuvastatin, pravastatin, orlistat and simvastatin stimulants: including amphetamine, phentermine, tyramine, ephedrine, metaraminol, phenylephrine, dexamphetamine, dexfenfluramine, fenfluramine, nicotine, caffeine and mazindol vasocontrictors: including desmopressin vasodilitors: including carvedilol, terazosin, phentolamine and menthol antialzheimers: including levetiracetam, levitiracetam and donepezil ACE inhibitors: including benzapril, enalapril, ramipril, fosinopril sodium, lisinopril, minoxidil, isosorbide, rampril and quinapril beta adrenoreceptor antogonists: including atenolol, timolol, pindolol, propanolol hydrochloride, bisoprolol, esmolol, metoprolol succinate, metoprolol and metoprolol tartrate angiotensin II antagonists: including losartan platelet inhibitors: including abciximab, clopidrogel, tirofiban and aspirin alcohols and phenols: including tramadol, tramadol hydrochloride, allopurinol, calcitriol, cilostazol, soltalol, urasodiol bromperidol, droperidol, flupenthixol decanoate, albuterol, albuterol sulphate, carisoprodol, chlobetasol, ropinirol, labetalol, and methocarbamol ketones and esters: including amioderone, fluticasone, spironolactone, prednisone, triazodone, desoximetasone, methyl prednisdone, benzonatate nabumetone and buspirone antiemetics: including metoclopramide ocular treatments: including dorzolamide, brimonidine, olopatadine, cyclopentolate, pilocarpine and echothiophate anticoagulant and antithrombitic agents: including warfarin, enoxaparin and lepirudin treatments for gout: including probenecid and sulfinpyrazone COPD and asthma treatments: including ipratropium treatments for osteoporosis: including raloxifene, pamidronate and risedronate cosmetic peptides: including acetyl hexapeptide-3, acetyl hexapeptide-8, acetyl octapeptide and I-carnosine vaccines: including vaccines comprising toxoids (inactivated toxic compounds); proteins, protein subunits and polypeptides; polynucleotides such as DNA and RNA; conjugates; adjuvants such as saponins, virosomes, inorganic and organic adjuvants, for example zostavax nutraceutical and cosmeceutical actives: including coenzyme Q 0 (or ubiquinone), ubiquinol or resveratrol; a carotenoid such as [alpha], [beta], or [gamma]-carotene, lycopene, lutein, zeaxanthin and astaxanthin; a phytonutrient, such as lycopene, lutein and seaxanthin; an unsaturated fatty acid such as linoleic acid, conjugated linoleic acid, linolenic acid, omega-3 fatty acids including but not limited to docosahexaenoic acid (DHA) and eicosapentaeonic acid (EPA) and their glycerol-esters; fat-soluble vitamins including vitamin D (D2, D3 and their derivatives), vitamin E ([alpha], [beta], [gamma], [delta]-tocopherols, or a, [beta], [gamma], [delta]-tocotrienols), vitamin A (retinol, retinal, retinoic acid and derivatives), vitamin K (K-i, K2, K3 and their derivatives) capric/caprylic triglycerides, folic acid, iron, niacin, glyceryl linoleate, omega 6 fatty acids, vitamin F, selenium, cyanocobalamin, aloe vera, beta glucan, bisabolol, camellia thea (green tea) extract, capric/caprylic triglycerides, centella asiatica (gotu cola) extract, cetearyl olivate, chlorophyll, citrus sinensis (orange) oil, cocoyl proline, dicapryl ether, disodium lauriminodipropionate tocopheryl phosphates (vitamin E phosphates), glycerin, glyceryl oleate, glycyrrhiza glabra (licorice) root extract, hamamelis virgiana (witch hazel) extract, lactic acid, lecithin, lutein, macadamia integrifolia (macadamia) seed oil, matricaria chamomilla (chamomile) extract, Oenothera biennis (evening primrose) oil, olea europaea (olive) leaf extract, rice bran oil, persea gratissima (avocado) oil, polygonum multiflorum extract, pomegranate sterols, resveratrol, rosa eglanteria (rose hip) oil, santalum spicatum (sandalwood) oil, titanium dioxide, folic acid, glycerin, glyceryl linoleate (omega 6 fatty acids vitamin F), vitamin A palmitate, vitis vinifera (grapeseed) oil, halobetasol, adenosine, adenosine triphosphate, alpha hydroxy acid, allantoin, hyaluronic acid and derivatives, isolutrol, tranexamic acid, glycolic acid, arginine, ascorbyl glucosamine, ascorbyl palmitate, salicylic acid, carnosic acid, alpha lipoic acid, gamma linolenic acid (GLA), panthenol, retinyl propionate, retinyl pamitate, furfuryladenine, retinaldehyde, copper pepetides, idebenone, dimethylaminoethanol (DMAE), niacinamide, beta- glucan, palmitoyl pentapeptide-4, palmitoyl oligopeptide/ tetrapetide-7, ethocyn, ceramides, phenylalanine, glucuronolactone, L-carnitine, hydroxylapetite, palmitoyl tripetide-3, forskolin, zinc oxide, α-bisabolol, eugenol, silybin, soy isoflavones, aucubin, catalpol, pseudoguaianolide from Arnica chamissonis, rosmarinic acid, rosmanol, salicylates for example salicin, saligenin and salicyclic acid, taxasterol, α-lactucerol, isolactucerol, taraxacoside, ceremides, arbutin, gingerols, shagaols, hypercin, elastin, collagen and peptides thereof.

The active pharmaceutical ingredient may be incorporated in any form, i.e. as such, in salt form, as a hydrate or solvate, in crystalline form, amorphous form, liquid (solution) form or in a vehicle such as micelles, liposomes, microparticles and the like.

The active pharmaceutical ingredient may be incorporated into the adhesive layer and/or it may be incorporated into a reservoir layer that is interposed between adhesive layer and backing layer.

The relative amount of active pharmaceutical ingredient is not particularly limited. It is also possible to incorporate two or more active pharmaceutical ingredients in the same or separate layers.

In the context of the present invention, it is possible to adjust the release rate of the active pharmaceutical ingredient by adjusting the hydrophilicity of the adhesive layer.

### 5.4.3 Sterilization

The medical tape or patch of the present invention can also be sterilized if the final application requires it. In a preferred embodiment the sterilization is performed by means of X-ray irradiation or γ-irradiation. Both those methods are compatible with the adhesive of the present invention and do not affect its properties. Of course, the medical tape or patch of the present invention may also be sterilized by other methods known in the art, such as wet heat, e-beam, ethylene oxide, peroxide, and the like.

### 5.5 Preferred Embodiment

The above general description describes preferred embodiments for most if not all of the above-mentioned features (monomers, other components, process conditions, etc.). Naturally, it is even more preferred to work the present invention by combining two or more of these preferred features. Similarly, if features are described as being more preferred or most preferred, it is even more preferred to combine these features with other features that are described as being preferred, more preferred, or most preferred. All feature combinations resulting from such an approach are meant to be disclosed as part of the present invention.

A particularly preferred subset of embodiments of the present invention is characterized by one or more of the appended claim 1 and additionally by the features described below:

According to the broadest first aspect of this subset, the polymer (polycondensate) is derived from polycondensation of a fatty acid dimer, and a fatty alcohol dimer (wherein the meaning and scope of these monomers is as specified in the general description above, including preferred and more preferred features). More preferably, in the second aspect, the features of the first aspect of this subset of preferred embodiments are combined with the feature of a molar ratio of OH groups to COOH groups before polycondensation being in the range of from 1.04 to 1.06. Even more preferable is a third aspect, wherein the features of the above first aspect and the feature of the second aspect are combined with the feature of a weight average molecular weight being in the range of from 50,000 to 62,000 g/mol (and even better: a range of from 53,000 to 59,000 g/mol). According to a fourth particularly preferred aspect of this subset of embodiments, the features of the first, second and third aspect are combined with the feature of using a mixture of isosorbide diesters as the oil component with the weight ratio of polycondensate to oil component being in the range of from 2:1 to 1:2. According to a fifth particularly preferred aspect of this subset of embodiments, the features of the first, second, third and fourth aspect are combined with the feature of using an aliphatic polyisocyanate crosslinking agent, such as the commercial product DESMODUR N3600. For polyurethane or polyethylene backings, the crosslinking agent is used in a relative amount of from 3.5 mol% to 4.5 mol% (or even better in a relative amount of from 3.75 mol% to 4.25 mol%), wherein mol% indications characterize the ratio of the molar amount of cross-linking agent divided by the molar amount of polycondensate, wherein the molar amounts are calculated taking (weight average) molecular weights of crosslinker and polycondensate into account. For nonwoven backings, the crosslinking agent is used in a relative amount of from 4.5 mol% to 9 mol%. Of course, all of the features described in connection with one or more of the above particularly preferred first, second, third, fourth and fifth embodiment of the present invention may be additionally combined or further characterized by one or more of those features of the above general description, which are fully consistent with the presently described particularly preferred embodiments.

### 5.6 Examples

### Preparative Example 1: Polycondensation of dimer diol with the dimer diacid (high molecular weight)

150.15 g of Pripol 1009 and 149.85 g of Pripol 2033 were loaded in a 500 mL reactor equipped with a vacuum adaptor and a mechanical stirring system (using an "anchor" shaped stirrer) and mixed at 150 - 220 rpm. Next 15 mg of Ti(OⁿBu)₄ was added to the reaction mixture and the colorless viscous solution was heated at 180ºC under vacuum (pressure ≤ 10 mbar) and kept under these conditions for 2h00 after which the heating temperature was set at 225ºC for 3h00 (pressure ≤ 5 mbar). Subsequently the pale yellow reaction mixture was allowed to cool under vacuum; once its temperature reached 130-140ºC, the reaction vessel was vented and discharged. Polycondensate A is obtained in this manner.

### Preparative Example 2: Polycondensation of dimer diol with the dimer diacid (medium molecular weight)

Polycondensate B is obtained in accordance with preparative example 1, but starting from 143.20 g of Pripol 1009 and 156.80 g of Pripol 2033.

### Preparative Example 3: Polycondensation of dimer diol and PEG with the dimer diacid

105.81 g of Pripol 1006, 107.11 g PEG (M_{w} 400) and 87.08 g of Pripol 2033 were loaded in a 500 mL reactor equipped with a vacuum adaptor and a mechanical stirring system (using and "anchor" shaped stirrer) and mixed mixed at 150 - 220 rpm. Next 50 mg of Ti(OⁿBu)₄ was added to the reaction mixture and the colorless viscous solution was heated at 180ºC under vacuum (pressure ≤ 10 mbar) and kept under these conditions for 2h00 after which the heating temperature was set at 225ºC for 4h00 (pressure ≤ 5 mbar). Subsequently the pale yellow reaction mixture was allowed to cool under vacuum; once its temperature reached 130-140ºC, the reaction vessel was vented and discharged. Polycondensate C is obtained in this manner.

### Preparative Example 4: Polycondensation of 1,4-Butonediol with the dimer diacid

255.21 g Pripol 1009 and 44.79 g 1,4-butanediol were loaded in a reactor equipped with a nitrogen inlet, a mechanical stirring system (using and "anchor" shaped stirrer) and a Dean and Stark apparatus mounted with a reflux condenser. 13 mg of Ti(OⁿBu)₄ was added and the viscous colorless solution was stirred at mixed at 150 - 220 rpm and heated at 180ºC under nitrogen stream and kept under these condition for 15h00 during which the solution slowly turns pale yellow. Next the heating was stopped and the reaction mixture was allowed to cool to 100-110ºC; the Dean and Stark apparatus was removed and replaced by a glass stopper. The nitrogen inlet was replaced by a vacuum adaptor and a second crop of Ti(OⁿBu)₄ (13 mg) was then introduced. Vacuum was applied (≥ 6 mbar) and heating was resumed (180ºC). After 2h00 at this temperature, the temperature was progressively increased to 250ºC. Next the now dark yellow-orange reaction mixture was allowed to cool (under vacuum), and once the its temperature reached 140-150ºC, the reaction vessel was vented and discharged. Polycondensate D is obtained in this manner.

**Table 1. Summary of the polymer properties (typical values).**

| Polycondensate | Mn | Mw | PDI | α transition |
|---|---|---|---|---|
| A | 30,998 | 56,894 | 1.83 | - 50°C |
| B | 21,808 | 30,696 | 1.40 | - 49°C |
| C | 21,169 | 33,612 | 1.59 | - 39°C |
| D | 42,512 | 82,051 | 1.92 | - 49°C |

### Preparative Example 5: Typical curing protocol using isocyanate crosslinker and an oil additive

10 g of polycondensate, 10 g of Polysorb ID 37 and 0.4 g of DESMODUR N3600 (4 weight %) were mixed together at room temperature. The obtained clear viscous paste was manually homogenized and then left on standing until disappearance of the air bubbles (∼ 60 min). Next the mixture was roll-coated manually at room temperature onto a PET liner and cured for 5 min at 130 ºC. The film obtained was laminated, on its "free" side, onto a protective film.
Adhesive 1: Polycondensate A is cured in accordance with preparative example 5 using 2.5 weight % (with regard to polycondensate) of DESMODUR N3600 as crosslinker and 100 weight % of Polysorb ID 37^{®} (Roquette Frères, France).
Adhesive 2: Polycondensate B is cured in accordance with preparative Example 5 using 4.5 weight % (with regard to polycondensate) of DESMODUR N3600 as crosslinker and 60 weight % of Polysorb ID 37^{®} (Roquette Frères, France).
Adhesive 3: Polycondensate C is cured in accordance with preparative example 5 using 4.0 weight % (with regard to polycondensate) of DESMODUR N3600 as crosslinker and 100 weight % of Polysorb ID 37^{®} (Roquette Frères, France).
Adhesive 4: Polycondensate D is cured in accordance with preparative example 5 using 3.5 weight % (with regard to polycondensate) of DESMODUR N3600 as crosslinker and 60 weight % of Polysorb ID 37^{®} (Roquette Frères, France).

Comparative Example 1: Polycondensate A is cured in accordance with preparative example 5 using 1.5 weight % (with regard to polycondensate) of DESMODUR N3600 as crosslinker but no oil additive. Comparative Example 2: Commercial acrylic skin adhesive, namely Transpore^{®} (3M, USA). Comparative Example 3: Commercial silicone adhesive, namely Kindly Removal Silicone Tape^{®} (3M, USA).

### Adhesive tensile test

The stress-strain curve of the adhesive samples were recorded by measuring the amount of deformation at distinct intervals (stretching rate of 300mm/min). The maximum stress and maximum strain were extracted from stress-strain curve.

**Table 2. Tensile test measurement on adhesive made of polycondensate B crosslinked in accordance with preparative example 5 with DESMODUR N3600 (4% in mass with regard to the polycondensate content) and containing different oil at a fix loading of 50% in mass with regard to the polycondensate content.**

| Oil | Max. stress (N/mm²) | Max. Strain (%) |
|---|---|---|
| No oil | 0.898 | 958 |
| Soybean oil | 0.100 | 1089 |
| Oleic acid | 0.039 | 1410 |
| Ethyl oleate | 0.205 | 985 |
| POLYSORB ID 37 | 0.115 | 1183 |

### Peel Test on porcine skin

Fresh porcine ears were enrolled into this study. The ears were washed by water gently and wiped with paper towel softly. After drying them for 2 hours, the ears skin is pre-stripped by Transpare^{®} (3M, USA). The pre-stripping tapes were cut in slices of 25 mm width and 70mm length. The tapes were secured using a 2 kg roller and allowed for 15 min before removal. The tapes were peeled off the subject, which is immobilized by flame, at 180 degrees at a rate of 300mm/min. This pre-stripping process was repeated twice. The adhesive samples were cut in slices of 25 mm width and 60 mm length. Samples were placed on the subject (the spot of application of a given sample was the same area of pre-stripping). The sample materials were secured using a 2 kg roller and allowed for 30 min before removal. The samples were peeled off the subject at 180 degrees at a rate of 300mm/min. The peel force was measured using a Shimadzu Ag-X universal tensile testing machine.

**Table 3. Summary of the adhesive properties on porcine skin (typical values).**

| | Peel Force [N/25mm] | Surface Tension [mN/m] |
|---|---|---|
| Adhesive 1 | 1.33 | 33 |
| Adhesive 2 | 0.35 | 33 |
| Adhesive 3 | 0.40 | 33 |
| Comparative Example 1 | 3.79 | 39 |
| Comparative Example 2 | 1.65 | 57 |
| Comparative Example 3 | 1.34 | 33 |

### Cell Removal evaluation

A removed adhesive tape from porcine skin was soaked in paraformaldehyde solution in PBS for 30 min. Next it was rinsed with distilled water, and the tape was incubated with 1% eosin solution for 9 min in order to stain it. Then the tape was washed by distilled water and observed by microscope.

**Table 4. Summary of the adhesive cell removal properties on porcine skin (typical values).**

| | Cell Removal Area [%] | |
|---|---|---|
| | Average | Standard deviation |
| Adhesive 1 | 15.0 | 5.5 |
| Adhesive 2 | 25.7 | 16.3 |
| Adhesive 3 | 10.1 | 1.2 |
| Comparative Example 1 | 64.5 | 15.0 |
| Comparative Example 2 | 62.5 | 11.0 |
| Comparative Example 3 | 12.6 | 7.7 |

A graphical representation of these results is given in Figure 1.

## Claims

1. Adhesive composition comprising
a cross-linked polycondensate (A); and
an oil additive (B);
wherein the cross-linked polycondensate contains repeating units derived from
a dicarboxylic acid (a) and
a diol (b);
wherein the weight average molecular weight of the polycondensate (A) before cross-linking is from 20,000 g/mol to 85,000 g/mol; and
wherein the content of oil additive (B) is 1 wt% to 150 wt% more preferably 80 wt% to 120 wt%, relative to the cross-linked polycondensate (A).

2. Adhesive composition according to claim 1, wherein a tackifier is additionally present at a concentration from 0.1 wt% to 30 wt% more preferably 1 wt% to 5 wt%, relative to the weight of the polycondensate.

3. Adhesive composition according to claim 1 or 2, wherein the dicarboxylic acid (a) is a dicarboxylic acid selected from general formula (I),
R^{a}OOC-R¹-R²-R³-COOR^{b} (I)
wherein R¹ is absent or selected from C₁₋₁₅ alkylene, C₂₋₁₅ alkenylene, and C₂₋₁₅ alkynylene, wherein each of these groups is optionally substituted; R² is selected from C₁₋₁₂ alkylene, C₂₋₁₂ alkenylene, and C₂₋₁₂ alkynylene, each of which may contain one or two 5-7-membered rings, which may optionally be condensed wherein each of these groups is optionally substituted; R³ is absent or selected from C₁₋₁₅ alkylene, C₂₋₁₅ alkenylene, and C₂₋₁₅ alkynylene wherein each of these groups is optionally substituted; each of R^{a} and R^{b} is independently selected from hydrogen, alkyl groups having 1 to 24 carbon atoms, and glycerol.

4. Adhesive composition according to claim 3, wherein the dicarboxylic acid (a) is a dimer of fatty acids.

5. Adhesive composition according to claim 1, 2, 3 or 4, wherein the diol (b) is a diol selected from general formula (II),
HO-R⁴-R⁵-R⁶-OH (II)
wherein R⁴ is absent or selected from C₁₋₁₅ alkylene, C₂₋₁₅ alkenylene, and C₂₋₁₅ alkynylene, wherein each of these groups is optionally substituted; R⁵ is selected from C₁₋₁₂ alkylene, C₂₋₁₂ alkenylene, and C₂₋₁₂ alkynylene, each of which may contain one or two 5-7-membered rings, which may optionally be condensed, wherein each of these groups is optionally substituted;
R⁶ is absent or selected from C₁₋₁₅ alkylene, C₂₋₁₅ alkenylene, and C₂₋₁₅ alkynylene, wherein each of these groups is optionally substituted.

6. Adhesive composition according to claim 5, wherein the diol (b) is a dimer of fatty alcohols.

7. Adhesive composition according to any one of claims 1 to 6, wherein the oil additive is selected from sorbitan mono- or di-esters of fatty acids, PEG diester, sesame oil, isosorbide diester, mixtures of isosorbide diesters, sorbitol mono- and diesters, oleate esters as well as vernonia oil or mixtures thereof.

8. Adhesive composition according to any one of claims 1 to 7, wherein the cross-linked polycondensate (A) has a gel content value between 60 % and 95%, preferably between 70 % and 80%.

9. Method for producing the adhesive composition according to any one of claims 1 to 8, which comprises the steps of
(i) Providing a polycondensate (A) as specified in one or more of claims 1 and 3 to 6;
(ii) Providing a cross-linking agent;
(iii) Providing an oil additive as specified in one or more of claims 1 and 7;
(iv) Mixing the ingredients of steps (i), (ii) and (iii) in any relative order;
(v) Subjecting the mixture of step (iv) to cross-linking reaction conditions.

10. Method for producing the adhesive composition according to claim 9, wherein step (i) comprises the further steps of
(ia) Providing a dicarboxylic acid (a) as specified in one or more of claims 1, 3 and 4;
(ib) Providing a diol (b) as specified in one or more of claims 1, 5 and 6;
(ic) Mixing the ingredients of steps (ia) and (ib); and
(id) Subjecting the mixture of step (ic) to polycondensation conditions.

11. Medical tape or patch comprising an adhesive layer made from the adhesive composition of any one of claims 1 to 8, which is formed on a backing.

12. Method for producing the medical tape or patch according to claim 11, which comprises the steps of
(va) Providing a mixture of ingredients obtainable according to steps (i) to (iv) of claim 9.
(vb) Coating the mixture of step (va) onto a backing;
(vc) Subjecting the mixture of step (vb) to cross-linking reaction conditions.

13. Method for producing the medical tape or patch according to claim 11, which comprises the steps of
(va') Providing a mixture of ingredients obtainable according to steps (i) to (iv) of claim 9.
(vb') Coating the mixture of step (va') onto a liner;
(vc') Subjecting the mixture of step (vb') to cross-linking reaction conditions to yield an adhesive;
(vd') Laminating the free side of the adhesive onto a backing, support or protective film.
